# FASCICULE DE BREVET EUROPEEN

(11) **EP 2 296 764 B1**
(45) Date de publication et mention de la délivrance du brevet: **10.08.2016**
(21) Numéro de dépôt: 09766057.5
(22) Date de dépôt: 26.05.2009
(51) Int. Cl.: A01N 25/16, A01N 25/22, A01N 25/30, A01N 37/16, A61L 2/18, C06B 21/00, A62D 3/38, A62D 101/02, A62D 101/04

(54) **SOLUTION AQUEUSE DECONTAMINANTE ET MOUSSANTE**
SCHÄUMENDE UND DEKONTAMINIERENDE WÄSSRIGE LÖSUNG
FOAMING DECONTAMINATING AQUEOUS SOLUTION

(30) Priorité: 27.05.2008 FR 0853437
(43) Date de publication de la demande: 23.03.2011
(73) Titulaire: Commissariat à l'Énergie Atomique et aux Énergies Alternatives, 75015 Paris (FR)
(72) Inventeur: GOFFINET, Pierre, F-91190 Gif-sur-Yvette (FR)
(74) Mandataire: Nevant, Marc
(86) Numéro de dépôt international: PCT/FR2009/050973
(87) Numéro de publication internationale: WO 2009/153495

(56) Documents cités:
- WO-A-00/51687
- WO-A-2005/070205
- FR-A- 2 766 724
- US-A- 2 392 936
- US-A1- 2003 060 517
- US-A1- 2005 239 675
- US-B1- 6 376 436
- DATABASE WPI Week 200674 Thomson Scientific, London, GB; AN 2006-712297 XP002516301 & JP 2006 257348 A (KAO CORP) 28 septembre 2006 (2006-09-28)

## Description

La présente invention est relative à la décontamination de surfaces ou la destruction d'explosifs susceptibles de contenir des agents toxiques chimiques ou bactériologiques, en particulier des agents toxiques et/ou polluants organophosphorés ou organosoufrés, notamment dans le domaine des agents de guerre chimique ou dans le domaine de l'agriculture, par exemple pour la décontamination d'insecticides.

Il existe actuellement de nombreux esters organophosphorés dérivés des acides phosphoriques et phosphoniques, utilisés comme agents de guerre chimique ou toxiques de guerre tels que les toxiques ou produits G : le "Tabun", le "Sarin" ou le "Soman", ou en tant qu'insecticides dans l'agriculture, comme par exemple le "paraoxon", le "diazinon" ou le toxique VX.

Ces composés présentent une importante neurotoxicité du fait de leur pouvoir phosphorylant vis-à-vis des cholinestérases dont l'inhibition entraîne la mort par accumulation d'acétylcholine dans l'organisme.

Par ailleurs, il existe également des produits toxiques organosoufrés, tels que les sulfures industriels ou les agents de guerre de la famille des vésicants, tels que l'ypérite (ou toxique HD).

L'existence de stocks importants de munitions de guerre susceptibles de contenir des agents toxiques chimiques ou bactériologiques et la menace croissante d'actions terroristes utilisant des systèmes explosifs ou dispersants contenant de tels agents toxiques ont suscité des recherches de solutions efficaces de décontamination et/ou destruction.

En règle générale, la décontamination des agents toxiques ou bactériologiques est effectuée par réduction, oxydation ou hydrolyse.

Il existe plusieurs types de compositions décontaminantes. On peut citer par exemple les solutions alcalines, telles que les solutions de soude, d'alcanolamines ou d'amines ou les solutions d'hypochlorites. Ces solutions ont l'inconvénient d'être très agressives vis-à-vis des matériaux contaminés.

D'autres compositions contenant un agent peroxydant organique ou minéral, tel qu'un peracide ou un peroxyde ont été préconisés, éventuellement en combinaison avec un agent tensio-actif de type ammonium quaternaire (FR 2 651 133 et FR 2 676 368), un agent séquestrant (FR 2 766 725) ou un agent tensioactif constitué par un oxyde d'amine (FR 2 766 724).

On a également décrit des compositions décontaminantes contenant des dérivés chlorés tels que les chloroisocyanurates (WO 00/51687 and DE 1 005 899) ; des biocides incluant des dérivés d'ammonium quaternaire en combinaison avec des enzymes (WO 01/0056380) ou des nanoparticules d'hydroxydes et d'oxydes métalliques notamment de métaux alcalins, alcalinoterreux et de métaux de transition (US 6 827 766).

Il a été proposé d'ajouter un agent moussant dans ces compositions décontaminantes (US 6 376 436 et US 2 392 936).

Dans de nombreux cas, il est hautement souhaitable de faire exploser sur place le système explosif ou dispersant en utilisant un détonateur ou une contre-charge.

Afin de limiter les effets du souffle de l'explosion, on a déjà proposé d'utiliser une mousse capable d'absorber la pression générée par l'onde de choc de l'explosion (WO 94/00198). Le plus souvent, lorsque l'explosif ne peut pas être transporté, il est confiné dans une enceinte mobile (WO 98/56465) telle que par exemple une tente en Kevlar^{®} dont l'espace intérieur est intégralement rempli d'une mousse obtenue in situ par diffusion d'un courant gazeux dans une solution aqueuse d'une composition contenant un agent moussant, tel qu'un agent tensio-actif (WO 94/00198).

Ces compositions moussantes sont généralement formulées à base d'agents tensio-actifs anioniques. En effet, ces agents tensio-actifs anioniques permettent d'obtenir des coefficients de foisonnement élevés, une excellente stabilité et un comportement viscoélastique de la mousse favorables à l'absorption de l'énergie du souffle de l'explosion ou « blast ».

Il est souhaitable de pouvoir disposer de compositions moussantes qui soient également décontaminantes afin de pouvoir assurer simultanément la destruction de l'explosif et la décontamination des agents toxiques qu'il est susceptible de contenir.

Compte tenu de la nature des réactions chimiques mises en jeu pour assurer la décontamination des agents toxiques chimiques (oxydation ou substitution nucléophile), les agents décontaminants sont généralement mis en oeuvre en présence d'agents tensio-actifs cationiques. Les agents toxiques sont décomposés par une réaction de catalyse micellaire par l'intermédiaire de microémulsions des agents décontaminants qui se forment en présence de l'agent tensio-actif cationique. De plus, la combinaison d'agents tensio-actifs cationiques et de peracides présente un très large spectre d'efficacité décontaminante sur les agents biologiques (bactéries, virus, spores et toxines). Malheureusement, les agents tensio-actifs cationiques qui ont une concentration micellaire critique élevée ne sont pas, par eux-mêmes, de très bons agents moussants. De plus, leur présence dans des compositions moussantes comprenant des agents tensio-actifs anioniques conduit à la formation d'un complexe électro-neutre hydrophobe très peu soluble en milieux aqueux et à la déstabilisation de la mousse.

Le demandeur a maintenant trouvé une nouvelle solution décontaminante exempte d'agents tensio-actifs cationiques qui permet à la fois de réduire l'effet de souffle d'un engin explosif et d'assurer la décontamination des agents toxiques et/ou bactériologiques contenus dans celui-ci.

Ainsi, la présente invention a pour objet une solution aqueuse décontaminante et moussante qui comprend, en poids, par rapport au poids total de la composition :
- 0,1 à 20 %, de préférence 0,3 à 5 %, d'un agent peroxydant hydrosoluble organique ou minéral tel que défini ci-après ;
- 0,05 à 20 %, de préférence 0,1 à 5 %, d'un agent tensio-actif anionique ;
- 0,1 à 20%, de préférence 0,1 à 5 %, d'un oxyde d'amine ;
- 0 à 5%, de préférence 0,1 à 2%, d'un agent stabilisant de l'agent peroxydant ;
- 0 à 10%, de préférence 0,5 à 4%, d'un agent tensio-actif amphotère ;
- 0 à 15%, de préférence 0 à 10%, d'un tampon alcalin ;
- le complément à 100% étant de l'eau ;
le pH de ladite solution aqueuse étant compris entre 7 et 11, de préférence entre 8,0 et 9,5.

L'invention a également pour objet la composition pour la préparation de la solution aqueuse décontaminante et moussante de l'invention.

Cette composition contient tous les constituants nécessaires pour la préparation de la solution aqueuse selon l'invention, et éventuellement, tout ou partie de l'eau.

De préférence, dans la composition pour la préparation de la solution aqueuse décontaminante et moussante de l'invention, l'agent peroxydant, et éventuellement son agent stabilisateur, sont séparés des autres constituants. Ainsi, de manière préférée, ladite composition comprend dans des conditionnements séparés I et II :
- l'agent peroxydant hydrosoluble organique ou minéral et, éventuellement, un agent stabilisateur dudit agent peroxydant hydrosoluble dans le conditionnement I ;
- les autres constituants et éventuellement tout ou partie de l'eau nécessaire pour préparer la solution aqueuse de l'invention dans le conditionnement II.

De préférence, le conditionnement II contient les constituants autres que l'agent peroxydant et son stabilisateur sous la forme de solution concentrée, la limite de concentration étant la limite de solubilité des constituants contenus dans le conditionnement II.

Avantageusement, le conditionnement II est dépourvu d'eau ou contient seulement la quantité d'eau nécessaire pour former une solution concentrée des autres constituants. La quantité d'eau à ajouter pourra aisément être déterminée par l'homme du métier, en fonction de la solubilité des autres constituants.

L'agent peroxydant hydrosoluble organique ou minéral est choisi parmi :
- les peracides organiques de formule générale R'-CO₃H dans laquelle R' est un groupe alkyle, linéaire ou ramifié, ayant de 1 à 4 atomes de carbone, de préférence de 1 à 3 atomes de carbone et leurs sels de métaux alcalins, alcalinoterreux, d'ammonium ou d'hydroxylamines ;
- les peracides organiques de formule générale HO₃C-R'-CO₃H dans laquelle R' est un groupe alkylène, linéaire ou ramifié, de 1 à 4 atomes de carbone, de préférence de 1 à 3 atomes de carbone et leurs sels de métaux alcalins, alcalinoterreux, d'ammonium ou d'hydroxylamines ;
- les peracides organiques de formule générale R"-(Φ) (CO₃H)ₙ(CO₂H)ₘ dans laquelle R" est un hydrogène ou un groupe alkyle, linéaire ou ramifié de 1 à 4 atomes de carbone, (Φ) est un noyau benzénique, n=1 ou 2, m=0 ou 1 et leurs sels de métaux alcalins, alcalinoterreux, d'ammonium ou d'hydroxylamines ;
- les peroxydes minéraux choisis parmi les adduits de H₂O₂, les persels, les peracides minéraux et leurs sels alcalins, notamment les parcarbonates, persulfates de sodium tels que le caroate de sodium (ou oxone), de potassium et d'ammonium, le peroxyde d'urée. Ces peroxydes sont optionnellement activés par un activateur de peroxydation choisi parmi la tétraacétyléthylènediamine (TAED), le pentaacétylglucose (PAG), le tétraacétylglucoluryle (TAGU), l'acide tétraacétylcyanurique (TACA), l'α-acétoxy-α-méthyl N,N'-(diacétyl)malonamide, l'acide acétylsallicylique (ASA), le paraacétoxybenzène sulfonate de sodium (AOBS), la diacétyl diméthylglyoxime (DDG), l'éthylidènebenzoacétate (EBA), l'anhydride phtalique (PAN), le benzoylimidazole (BID), le parabenzoxybenzènesulfonate de sodium (BOBS), le nonanoyloxybenzènesulfonate de sodium (NOBS) et l'isononanoyloxybenzènesulfonate de sodium (ISONOBS) ;
- Les mélanges desdits agents peroxydants.

A titre d'exemples d'agents peroxydants hydrosolubles préférés aux fins de l'invention, on peut citer notamment :
- l'acide peracétique, le peracétate de sodium, l'acide perpropionique, l'acide performique, le perpropionate de potassium ;
- le mono peroxyphtalate de magnésium hexahydraté (MPPM), l'acide perphtalique, l'acide perbenzoïque, etc.
- le caroate de sodium, le percarbonate de sodium, etc.

L'agent tensio-actif anionique peut être choisi parmi les agents anioniques ci-après :
- les alkyl-benzène-sulfonates solubles dans l'eau dont les groupes alkyle, à chaîne droite ou ramifiée, contiennent environ 8 à 16 atomes de carbone ;
- les alkyl-éther-sulfates ou alcényl-éther-sulfates solubles dans l'eau dont les groupes alkyle ou alcényle, à chaîne droite ou ramifiée, contiennent environ 10 à 20, de préférence 12 à 18, atomes de carbone et en moyenne de 0,5 à 8 moles, de préférence 2 à 4 moles, d'oxyde d'alkylène en C₂-C₄, par exemple d'oxyde d'éthylène, de propylène, de butylène ou un mélange de ces oxydes d'alkylène ;
- les alkyl-sulfates ou alcényl-sulfates solubles dans l'eau dont les groupes alkyle ou alcényle, à chaîne droite ou ramifiée, ont environ 10 à 22 atomes de carbone, de préférence 12 à 18 atomes de carbone ;
- les mono- ou di-alkyl- ou alcényl-sulfosuccinates solubles dans l'eau, dont les groupes alkyle ou alcényle, à chaîne droite ou ramifiée, ont environ 8 à 22 atomes de carbone ;
- les alkyl- ou alcényl-phosphates linéaires ou ramifiés solubles dans l'eau comportant de 8 à 22 atomes de carbone ;
- les savons d'acides gras saturés ou insaturés solubles dans l'eau comprenant de 8 à 22 atomes de carbone ;
- les acides gras alpha-sulfonés solubles dans l'eau comprenant de 8 à 22 atomes de carbone et leurs esters résultant de la condensation avec un alcool saturé ou insaturé comprenant de 1 à 10 atomes de carbone ou un groupe oxyde d'alkylène résultant de la condensation de 1 à 10 unités d'oxyde d'éthylène ou de propylène ;
- Les alkyltaurates et alkyliséthionates solubles dans l'eau dont la chaîne alkyle comprend entre 8 et 22 atomes de carbone ;
- les sulfates ou sulfonates de N-alkyl amides d'acides gras solubles dans l'eau comprenant de 8 à 22 atomes de carbone, l'atome d'azote portant de 0 à 2 atomes d'hydrogène et de 0 à 2 groupes alkyle saturés ou insaturés comprenant de 1 à 6 atomes de carbone ou un groupe résultant de la condensation de 1 à 10 unités d'oxyde d'éthylène ou de propylène ;
- les benzoylsulfopropionates d'alkyle solubles dans l'eau dont les groupes alkyle, à chaîne droite ou ramifiée, contiennent de 8 à 22 atomes de carbone ;
- les sulfonates ou sulfates d'esters d'acides gras solubles dans l'eau répondant à l'une des formules ci-après :

   R-C(=O)-O-R'-SO₃-X et R-C(=O)-O-R'-SO₄-X

   dans lesquelles R est un groupe alkyle à chaîne droite ou ramifiée ayant environ 8 à 22 atomes de carbone, R' est un groupe alkylène saturé ou insaturé comprenant de 1 à 6 atomes de carbone et X est, un cation métalliques alcalin, NH₄⁺ ou un cation d'alcanolamine en C₁-C₆ telle que la monoéthanolamine, la diéthanolamine, la triéthanolamine ou l'isopropanolamine ;
- les alkyl éthers sulfonés solubles dans l'eau de formule générale :

   R-O-R'-SO₃-X

   dans laquelle R est un groupe alkyle à chaîne droite ou ramifiée ayant environ 8 à 22 atomes de carbone, R' est un groupe alkylène saturé ou insaturé comprenant de 1 à 6 atomes de carbone et X est un cation métallique alcalin, NH₄⁺ ou un cation d'alcanolamine en C₁-C₆ telle que la monoéthanolamine, la diéthanolamine, la triéthanolamine ou l'sopropanolamine
- les mélanges desdits agents tensio-actifs anioniques.

Les agents tensio-actifs anioniques ci-dessus sont des agents anioniques disponibles dans le commerce ou qui peuvent être préparés par les procédés classiques bien connus de l'homme du métier. A titre de référence concernant ces agents tensio-actifs anioniques on citera les ouvrages ci-après :
* « Anionic surfactants » par M. LINFIELD (Coll. Surfactant Science Collection)
   - Editeur Warner;
* «Amphoteric Surfactants » par B.R. BLUESTEIN et al. (Coll. Surfactant Science Collection) - Editeur L. Hiltoy.

De préférence, on choisira un agent tensio-actif anionique qui permet une réduction de la tension superficielle de l'eau à 20°C d'au moins 4,0.10⁻²N/m. Le pouvoir moussant de la composition selon l'invention est déterminée selon la norme NF T 73404, dite également test de Ross-Miles.

La composition peut contenir de 0 à 10%, de préférence 0,5 % à 4% d'un agent tensio-actif amphotère tel que les (C₈-C₁₈)alkylbétaines; les (C₈-C₁₈) alkylsulfobétaines et les (C₈-C₁₈) alkylamphocarboxyglycinates ou les (C₈-C₁₈)alkylamphocarboxypropionates ou les mélanges de ceux-ci.

L'oxyde d'amine mis en oeuvre dans la composition décontaminante et moussante selon l'invention est un oxyde d'amine quelconque capable d'assurer la catalyse micellaire au sein de la composition. Les réactions d'oxydation et de substitution nucléophiles sont ainsi favorisées par l'augmentation de la surface d'échange entre la composition décontaminante et moussante de l'invention et le toxique.

La fonction de l'oxyde d'amine au sein de la composition selon l'invention est totalement différente de la fonction de stabilisation décrite dans GB 1089997.

On a constaté que l'utilisation d'un oxyde d'amine en tant que catalyseur micellaire à la place d'un agent tensio-actif cationique ne réduit pas, mais au contraire, renforce considérablement le pouvoir moussant de la composition décontaminante.

Avantageusement, l'oxyde d'amine sera choisi parmi :
- les oxydes d'amine répondant aux formules ci-après : 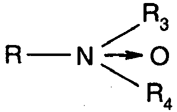 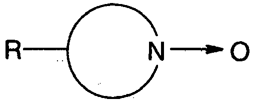 dans lesquelles :
   - R est un groupe alkyle ou alcényle, linéaire ou ramifié, contenant 8 à 22 atomes de carbone, éventuellement interrompu par un groupe -O-C(=O) ; -O- ; -O-NH₂- ou -O-NR₁R₂- ;
   - R₃ et R₄, identiques ou différents, représentent un alkyle en C₁-C₄ ou un groupe de formule [(CH₂)ₘ-O]ₙ-H dans laquelle m et n sont compris entre 1 et 4.
   - 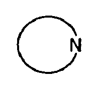 est un hétérocycle contenant 2 à 6 atomes de carbone et au moins un atome d'azote.
- Les mélanges desdits oxydes d'amine.

Les oxydes d'amine ci-dessus sont des oxydes d'amine disponibles dans le commerce ou qui peuvent être par les procédés classiques bien connus de l'homme du métier, par exemple par réaction d'oxydation des amines tertiaires par les agents peroxydants, par exemple, le peroxyde d'hydrogène. Des exemples d'oxyde d'amine répondant à l'un ou l'autre des formules ci-dessus sont :
- l'oxyde de N-lauryl-diméthylamine ;
- l'oxyde de lauryl-pyridine ;
- l'oxyde de N-cétyl-diéthylamine ;
- l'oxyde d'octyl-pyridine ;
- l'oxyde de N-myristyl-di-(2-hydroxyéthyl)amine.

On peut utiliser un tampon alcalin quelconque capable d'ajuster le pH de la composition de l'invention entre 7 et 11, de préférence entre 8,0 et 9,5.

Le tampon alcalin est de préférence choisi parmi les carbonates, les bicarbonates, les borates, silicates, citrates, tartrates, phosphates de métaux alcalins ou alcalino-terreux ou leurs mélanges.

Les tampons carbonate, borate, phosphate (ortho, pyro ou triplyphosphate), citrate de sodium ou de potassium sont particulièrement préférés en particulier les carbonates.

De plus, la composition de l'invention peut éventuellement comprendre un ou plusieurs agents stabilisateurs de l'agent peroxydant tel que :
- les acides et sels de phosphonates organiques, tels que l'acide 1-hydroxyéthylène-1,1-diphosphonique (HEDP), l'éthylène-diamine-tétraméthylène-phosphonate (EDTMP), l'acide diéthylènetriamine-pentaméthylène phosphonique (DTPMP) ;
- les acides et sels d'aminopolycarbonates, tels que l'acide éthylène-diamine-tétraacétique (EDTA) et l'acide diéthylène-triamine-pentaacétique (DTPA) ;
- l'acide citrique et ses sels ;
- la 8-hydroxyquinoléine ;
- l'acide dipicolinique et ses sels ;
- les traces de sels d'argent colloïdaux ;
- l'acide tartrique et ses sels ;
- des agents séquestrants pour capter les métaux di- ou tri-valents.

Optionnellement, la solution aqueuse selon l'invention peut également comprendre un agent tensio-actif non ionique de la famille des alcools gras et des alcools gras éthoxylés, des alcools gras éthoxylés-propoxylés, des amides gras, des amides gras éthoxylés, notamment les diéthanolamides, alkyle polyglucosides, etc ...

La solution aqueuse selon l'invention peut également comprendre des agents régulateurs de viscosité, tels que les gommes xanthane, carraghénane, gellane, guar, la Laponite^{®} (argile colloidale : silicate de Mg, Na et Li), les carboxyméthylcellulose, méthylcellulose, hydroxyéthylcellulose, hydroxypropylcellulose, l'amidon modifié, le polyéthylène glycol, l'acide polyacrylique et les polyacrylates (carbomers), etc ..., de préférence de masse moléculaire comprise entre 400 et 400 000 Dalton.

Enfin, la solution aqueuse selon l'invention peut contenir, toujours à titre optionnel, des solvants alcooliques ou glycoliques, des polyglycols, des éthers de glycol, des agents hydrotropes (urée, phénol éthoxylé ayant 3-6 moles d'oxyde d'éthylène, cumène sulfonates, toluène sulfonates ou xylène sulfonates de métaux alcalins ou d'ammonium).

Les solutions aqueuses selon l'invention sont obtenues par addition extemporanéé de l'agent peroxydant dans la solution aqueuse contenant les autres constituants.

L'invention a également pour objet le procédé de décontamination des surfaces qui consiste à :
1) générer une mousse à partir de la solution aqueuse selon l'invention ;
2) mettre en contact ladite mousse avec la surface à décontaminer pendant quelques minutes ;
3) récupérer les effluents après liquéfaction de la mousse.

L'invention a aussi pour objet le procédé de destruction d'explosifs susceptibles de contenir des agents toxiques chimiques ou bactériologiques qui consiste à :
1) générer une mousse à partir d'une solution aqueuse selon l'invention ;
2) confiner les explosifs à détruire dans une enceinte mobile ;
3) pulvériser dans ladite enceinte la mousse obtenue à l'étape 1) ;
4) mettre à feu lesdits explosifs à détruire ;
5) récupérer les effluents après liquéfaction de la mousse.

L'efficacité décontaminante et le pouvoir moussant des solutions aqueuses selon l'invention sur des toxiques de guerre a été évaluée sur des similis de toxiques, à savoir les similis de l'ypérite du VX et des produits G.

Ces similis de toxiques sont des molécules présentant un moindre degré de toxicité, mais une structure chimique et une réactivité démontrée comme équivalente à celle des toxiques réels.

### Simili de l'ypérite (HD) :

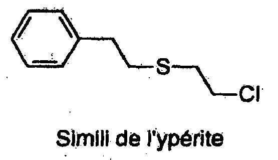

Ce produit proposé comme simili est le chloroéthylphényléthylsulfure (à comparer à l'ypérite : ClCH₂CH₂SCH₂CH₂Cl), et caractérisé par :
- la présence d'un atome de soufre
- la présence d'un Cl en β de l'atome de soufre pour mimer la formation d'un sulfonium intermédiaire.

Comme l'ypérite, il se détruit généralement par oxydation; l'atome de soufre est oxydé en chlorosulfoxyde, puis en chlorosulfone; la vinylsulfone se forme en milieu basique selon le schéma réactionnel ci-après, dans lequel R=Phényléthyle:

[Oxydation] [Oxydation] [Base] RSCH₂CH₂Cl → RS(O)CH₂CH₂Cl → RS(O)₂CH₂CH₂Cl → RS(O)₂CH=CH₂

La réaction est suivie en chromatographie phase gazeuse/masse et chromatographie sur couche mince.

### Simili du VX :

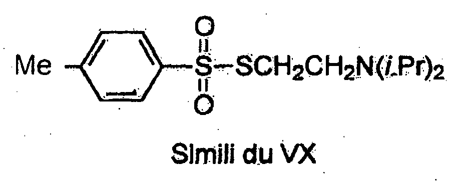

Ce produit, comme son homologue parafluoré est un bon simili du VX : CH₃CH₂-O-P(O)(CH₃)-S-CH₂CH₂-N(iPr)₂, notamment du fait de la présence de la fonction β-aminothiol. L'oxydation doit en effet porter sur le soufre et éviter la formation de N-oxyde (très toxique).
Comme le VX, il est détruit en milieu basique ou en présence d'un acide oxydant en donnant soit un disulfure, soit un acide sulfonique.
La réaction est suivie au cours du temps en CCM (révélation à l'iode).

### Simili des produits G :

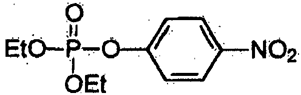

Paraoxon

Insecticide commercial ; excellent simili des produits G:
Ces produits se détruisent généralement par une réaction de substitution nucléophile du fluor (sarin, soman) ou du groupe cyano (tabun) 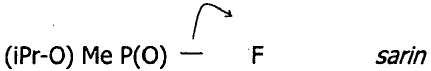 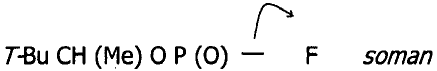 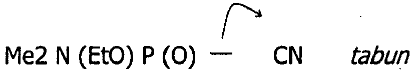 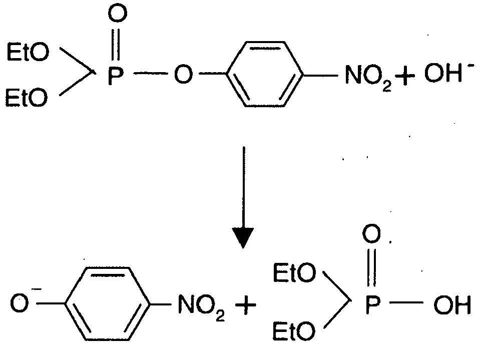 (paranitrophénate)

### La réaction est suivie :

- en chromatographie phase gazeuse (après extraction à l'acétate d'éthyle/hexane)

L'invention va maintenant être décrite plus en détail par les exemples illustratifs, mais non limitatifs ci-après.

### EXEMPLE 1 : Solution aqueuse décontaminante et moussante

Dans un récipient, on a mélangé l'agent peroxydant avec les autres constituants, et de l'eau, pour obtenir la solution aqueuse ci-après :

| | |
|---|---|
| - Acide peracétique (37 %) | 2,50 % |
| - Oxyde de N-lauryl diméthyl amine (30 %) | 3,00 % |
| - Na₂CO₃ | 3,50 % |
| - DTPMP de Na (30 %) | 4,00 % |
| - Laurylsulfate de sodium | 0,30 % |
| - Laurylsulfosuccinate d'éther d'alcool gras | 0,20 % |
| - Eau | 83,00 % |

Le pH de la solution était de 8,2.

### EXEMPLE 2 : Solution aqueuse décontaminante et moussante

Dans un récipient, on a mélangé l'agent peroxydant avec les autres constituants, et de l'eau, pour obtenir la solution aqueuse ci-après :

| | |
|---|---|
| - Acide peracétique (37 %) | 3,00 % |
| - Oxyde de N-lauryl pyridine | 2,00 % |
| - Disilicate de Na | 5,50 % |
| - HEDP de Na (30 %) | 1,00 % |
| - Lauryl éther sulfate de Na (28 %) | 8,00 % |
| - Eau | 80,50 % |

Le pH de la solution était de 9.

### EXEMPLE 3 : Solution aqueuse décontaminante et moussante

Dans un récipient, on a mélangé l'agent peroxydant avec les autres constituants, et de l'eau, pour obtenir la solution aqueuse ci-après :

| | |
|---|---|
| - MPPM | 12,00 % |
| - Oxyde de N-cétyl diéthyl amine | 7,00 % |
| - K₂CO₃ | 3,50 % |
| - Citrate de Na | 6,00 % |
| - Laurylsulfate de triéthanolamine | 0,30 % |
| - Eau | 68,50 % |

Le pH de la solution était de 8,5.

### Tests comparatifs

Dans ces tests on a comparé les solutions aqueuses selon l'invention décrites dans les exemples 1 à 3 avec la composition décontaminante classique A ci-après, dans laquelle on a ajouté la composition moussante B.

### Procédure de mesure de décontamination chimique sur similis de HP et VX

5 g de la solution aqueuse décontaminante et moussante de l'invention ont été déposés à l'aide d'une pipette sur une plaque de verre revêtue de similis de HD ou VX dans un sovirel.

On a agité pendant 20 minutes le sovirel, puis on a bloqué la réaction d'oxydation par 2 ml de thiosulfate.

On a procédé ensuite à l'extraction (simili et ses métabolites restant sur la plaque) par un mélange d'acétate d'éthyle/cyclohexane (50/50).

L'analyse de l'agent chimique (simili+métabolites) résiduel a été réalisée par CPG (colonne Silicone SE) ainsi que par chromatographie sur couche mince.

### Procédure de mesure de décontamination chimique sur simili de produits G

5 g de la solution aqueuse décontaminantë et moussante ont été déposés sur le simili de produit G dans un sovirel.

Le sovirel a ensuite été placé sur une plaque à secousse avec une agitation maximale pendant 20 min.

Dans le sovirel on a ensuite introduit :
- 10,0 ml de solution d'étalon interne (dibutylsébaçate) à 0,1% dans un mélange d'acétate d'éthyle/hexane (50/50).

Le sovirel a été rebouché et agité manuellement pendant 10 s.

La phase organique surnageante a été récupérée aussitôt dans un autre sovirel de 25 ml contenant du sulfate de Na et injectée en CPG sur colonner verre OV1.

Les résultats obtenus figurent dans le tableau I ci-après:

**Tableau I**

| **Pouvoir moussant (Test de Ross Miles - ISO 696)** | | | **Décontamination après 60 minutes** | | |
|---|---|---|---|---|---|
| **Solution** | Temps (en min) | Hauteur de mousse (en ml) | Simili de HD | Simili de VX | Simili de produit G (paraoxon) |
| | 0 | 470 | | | |
| | 3 | 415 | | | |
| | 5 | 400 | | | |
| **Exemple 1** | 10 | 400 | | | |
| | 20 | 400 | | | |
| | 30 | 390 | | | |
| | 60 | | 100% | 90% | 80% |
| | 0 | 410 | | | |
| | 3 | 390 | | | |
| | 5 | 380 | | | |
| **Exemple 2** | 10 | 380 | | | |
| | 20 | 380 | | | |
| | 30 | 380 | | | |
| | 60 | | 100 % | 90% | 90% |
| | 0 | 440 | | | |
| | 3 | 415 | | | |
| | 5 | 400 | | | |
| **Exemple 3** | 10 | 390 | | | |
| | 20 | 380 | | | |
| | 30 | 370 | | | |
| | 60 | | 90% | 80% | 70% |
| **Solution décontaminante A (17%) + solution moussante B (4%) + eau déminéralisée : qsp 100%** | 0 | 320 | | | |
| | 3 | 290 | | | |
| | 5 | 270 | | | |
| | 10 | 260 | | | |
| | 20 | 150 | | | |
| | 30 | 60 | | | |
| | 60 | | 30% | 40% | 40% |

| | **Matière** | **Fonction** | **% en masse** |
|---|---|---|---|
| **Solution décontaminante classique : A** | Monoperoxyphtallate de magnésium, 6 H₂O (MPPM) | Agent peroxydant | 47,0 |
| | Chlorure de myristalkonium | Catalyseur micellaire | 8,0 |
| | Na₂CO₃ | Tampon de pH | 18,0 |
| | NaOH | Alcalinisant | 3,0 |
| | Gluconate de sodium | Stabilisant | 24,0 |
| **Solution moussante : B** | Lauryl sulfate de triéthanolamine | Agent tensioactif anionique moussant | 12,0 |

## Revendications

1. Solution aqueuse décontaminante et moussante, **caractérisée en ce qu'**elle comprend, en poids par rapport au poids total de la composition :
- 0,1 à 20 % d'un agent peroxydant hydrosoluble organique ou minéral ;
- 0,05 à 20 % d'un agent tensio-actif anionique ;
- 0,1 à 20% d'un oxyde d'amine ;
- 0 à 5 % d'un agent stabilisateur de l'agent peroxydant ;
- 0 à 10 % d'un agent tensio actif amphotère ;
- 0 à 15 % d'un tampon alcalin ; et
- le complément à 100 % étant de l'eau ;
ledit agent péroxydant hydrosoluble organique ou minéral étant choisi parmi :
- les peracides organiques de formule générale R'-CO₃H dans laquelle R' est un groupe alkyle, linéaire ou ramifié, ayant de 1 à 4 atomes de carbone, de préférence de 1 à 3 atomes de carbone et leurs sels de métaux alcalins, alcalinoterreux, d'ammonium ou d'hydroxylamines ;
- les peracides organiques de formule générale HO₃C-R'-CO₃H dans laquelle R' est un groupe alkylène, linéaire ou ramifié, de 1 à 4, de préférence 1 à 3, atomes de carbone et leurs sels de métaux alcalins, alcalinoterreux, d'ammonium ou d'hydroxylamines ;
- les peracides organiques de formule générale R"-(Φ) (CO₃H)ₙ(CO₂H)ₘ dans laquelle R" est un hydrogène ou un groupe alkyle, linéaire ou ramifié de 1 à 4 atomes de carbone, (Φ) est un noyau benzénique, n=1 ou 2, m=0 ou 1, et leurs sels de métaux alcalins, alcalinoterreux, d'ammonium ou d'hydroxylamines ;
- les peroxydes minéraux choisis parmi les adduits de H₂O₂, les persels, les peracides minéraux et leurs sels alcalins ;
- les mélanges desdits agents peroxydants.
le pH de ladite solution aqueuse étant compris entre 7 et 11.

2. Solution selon la revendication 1, **caractérisée en ce que** le pH est compris entre 8,0 et 9,5.

3. Solution selon l'une des revendications 1 ou 2, **caractérisée en ce qu'**elle comprend, en poids par rapport au poids total de la composition :
- 0,3 à 5 % d'un agent peroxydant hydrosoluble organique ou minéral ;
- 0,1 à 5 % d'un agent tensio-actif anionique ;
- 0,5 à 4 % d'un agent tensio-actif amphotère ;
- 0,1 à 5 % d'un oxyde d'amine ;
- 0,1 à 2 % d'un agent stabilisateur de l'agent peroxydant ;
- 0 à 10 % d'un tampon alcalin ; et
- le complément à 100 % étant de l'eau ;

4. Solution selon l'une quelconque des revendications 1 à 3, **caractérisée en ce que** l'oxyde d'amine est choisi parmi : 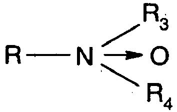 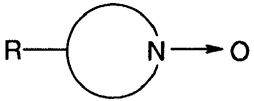 dans lesquelles :
- R est un groupe alkyle ou alcényle, linéaire ou ramifié, contenant 8 à 22 atomes de carbone, éventullement interrompu par un groupe -O-C(=O) ; -O- -O-NH₂- ou -O-NR₁R₂- ;
- R₃ et R₄, identiques ou différents, représentent un alkyle en C₁-C₄ ou un groupe de formule [(CH₂)ₘ-O]ₙ-H dans laquelle m et n sont compris entre 1 et 4.
- 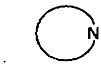 est un hétérocycle contenant 2 à 6 atomes de carbone et au moins un atome d'azote.

5. Solution selon la revendication 4, **caractérisée en ce que** l'oxyde d'amine est :
- l'oxyde de N-lauryl-diméthylamine ;
- l'oxyde de lauryl-pyridine ;
- l'oxyde de N-cétyl-diéthylamine ;
- l'oxyde d'octyl-pyridine ;
- l'oxyde de N-myristyl-di-(2-hydroxyéthyl)amine.

6. Solution selon la revendication 1, **caractérisée en ce que** l'agent peroxydant est :
- l'acide peracétique, le peracétate de sodium, l'acide perpropionique, l'acide performique, le perpropionate de potassium ;
- le mono peroxyphtalate de magnésium hexahydraté (MPPM), l'acide perphtalique, l'acide perbenzoïque.
- le caroate de sodium, le percarbonate de sodium.

7. Solution selon l'une quelconque des revendications 1 à 6, **caractérisée en ce que** l'agent tensio-actif anionique est choisi parmi les (C₁₂-C₁₈)alkylsulfates, les (C₁₂-C₁₈)alkyl-éther-sulfates ayant 2 à 4 moles d'oxyde d'alkylène en (C₂-C₄) les alkylsulfosuccinates, les (C₈-C₁₆) alkylbenzènsulfonates.

8. Composition pour la préparation d'une solution aqueuse décontaminante et moussante selon l'une quelconque des revendications 1 à 7.

9. Composition selon la revendications 8, **caractérisée en ce qu'**elle comprend, dans des conditionnements I et II séparés :
- l'agent peroxydant hydrosoluble organique ou minéral et, éventuellement, un agent stabilisateur de cet agent peroxydant hydrosoluble, dans le conditionnement I ;
- les autres constituants dans le conditionnement II, et éventuellement tout ou partie de l'eau, nécessaire pour la préparation de la solution aqueuse selon l'une quelconque des revendication 1 à 7.

10. Utilisation de la solution aqueuse selon l'une quelconque des revendications 1 à 7 pour la décontamination de systèmes dispersants et d'agents explosifs susceptibles de contenir des agents toxiques chimiques ou bactériologiques.

11. Procédé de décontamination de surfaces, **caractérisé en ce qu'**il consiste à :
1) générer une mousse à partir d'une solution aqueuse selon l'une quelconque des revendications 1 à 7 ;
2) mettre en contact ladite mousse avec la surface à décontaminer pendant quelques minutes ;
3) récupérer des effluents après liquéfaction de la mousse.

12. Procédé de destruction d'explosifs susceptibles de contenir des agents toxiques chimiques ou bactériologiques, **caractérisé en ce qu'**il consisite à :
1) générer une mousse à partir d'une solution aqueuse selon l'une quelconque des revendications 1 à 7 ;
2) confiner les explosifs à détruire dans une enceinte mobile ;
3) pulvériser dans ladite enceinte la mousse obtenue à l'étape 1) ;
4) mettre à feu desdits explosifs à détruire ;
5) récupérer des effluents après liquéfaction de la mousse.

## Patentansprüche

1. Dekontaminierende und schäumende wässerige Lösung, **dadurch gekennzeichnet, dass** sie in Gewicht bezogen auf das Gesamtgewicht der Zusammensetzung umfasst:
- 0,1 bis 20 % eines organischen oder mineralischen wasserlöslichen peroxidativen Wirkstoffes,
- 0,05 bis 20 % eines anionischen Tensids,
- 0,1 bis 20 % eines Aminoxids,
- 0 bis 5 % eines Stabilisators des peroxidativen Wirkstoffes,
- 0 bis 10 % eines amphoteren Tensids,
- 0 bis 15 % eines alkalischen Puffers, und
- wobei die Ergänzung auf 100 % Wasser ist,
wobei der mineralische oder organische wasserlösliche peroxidative Wirkstoff ausgewählt ist unter:
- den organischen Persäuren der allgemeinen Formel R'-CO₃H, wobei R' eine lineare oder verzweigte Alkylgruppe mit 1 bis 4 Kohlenstoffatomen, vorzugsweise 1 bis 3 Kohlenstoffatomen und ihren Salzen von alkalischen Metallen, erdalkalischen Metallen, Ammonium oder Hydroxylaminen ist,
- den organischen Persäuren der allgemeinen Formel HO₃C-R'-CO₃H, wobei R' eine lineare oder verzweigte Alkylengruppe mit 1 bis 4, vorzugsweise 1 bis 3, Kohlenstoffatomen und ihren Salzen von alkalischen Metallen, erdalkalischen Metallen, Ammonium oder Hydroxylaminen ist,
- den organischen Persäuren der allgemeinen Formel R"(Φ)(CO₃H)ₙ(CO₂H)ₘ, wobei R" ein Wasserstoff oder eine lineare oder verzweigte Alkylgruppe mit 1 bis 4 Kohlenstoffatomen ist, (Φ) ein Benzenkern ist, n=1 oder 2, m=0 oder 1, und ihren Salzen von alkalischen Metallen, erdalkalischen Metallen, Ammonium oder Hydroxylaminen ist,
- den mineralischen Peroxiden, die unter den H₂O₂-Addukten, den Pesalzen, den mineralischen Persäuren und ihren alkalischen Salzen ausgewählt sind,
- den Gemischen der peroxidativen Wirkstoffe,
wobei der pH-Wert der wässerigen Lösung zwischen 7 und 11 beträgt.

2. Lösung nach Anspruch 1, **dadurch gekennzeichnet, dass** der pH-Wert zwischen 8,0 und 9,5 beträgt.

3. Lösung nach einem der Ansprüche 1 oder 2, **dadurch gekennzeichnet, dass** sie in Gewicht bezogen auf das Gesamtgewicht der Zusammensetzung umfasst:
- 0,3 bis 5 % eines organischen oder mineralischen wasserlöslichen peroxidativen Wirkstoffes,
- 0,1 bis 5 % eines anionischen Tensids,
- 0,5 bis 4 % eines amphoteren Tensids,
- 0,1 bis 5 % eines Aminoxids,
- 0,1 bis 2 % eines Stabilisators des peroxidativen Wirkstoffes,
- 0 bis 10 % eines alkalischen Puffers, und
- wobei die Ergänzung auf 100 % Wasser ist.

4. Lösung nach einem der Ansprüche 1 bis 3, **dadurch gekennzeichnet, dass** das Aminoxid ausgewählt ist unter: 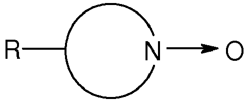 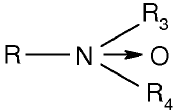 wobei:
- R eine lineare oder verzweigte Alkyl- oder Alkenylgruppe mit 8 bis 22 Kohlenstoffatomen ist, eventuell unterbrochen durch eine Gruppe -O-C(=O) -O-, --O-NH₂- oder -O-NR₁R₂-,
- wobei R₃ und R₄ identisch oder unterschiedlich sind, ein C₁-C₄-Alkly oder eine Gruppe der Formel [(CH₂)ₘ-O]ₙ-H darstellen, wobei m und n zwischen 1 und 4 betragen,
- 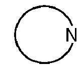 ein Heterozyklus ist, der 2 bis 6 Kohlenstoffatome und mindestens ein Stickstoffatom enthält.

5. Lösung nach Anspruch 4, **dadurch gekennzeichnet, dass** das Aminoxid ist:
- N-Lauryl-Diemthylamin-Oxid,
- Lauryl-Pyridin-Oxid,
- N-Ketyl-Diethylamin-Oxid,
- Octyl-Pyridin-Oxid,
- N-Myristyl-di-(2-Hydroxyethyl)amin-Oxid.

6. Lösung nach Anspruch 1, **dadurch gekennzeichnet, dass** der peroxidative Wirkstoff ist:
- Peressigsäure, Natriumperacetat, Perpropionsäure, Perameisensäure, Kaliumperpropionat,
- Magnesium-Hexahydrat-Mono-Peroxyphthalat (MPPM), Perphthalsäure, Perbenzoesäure,
- Natriumcaroat, Natriumpercarbonat.

7. Lösung nach einem der Ansprüche 1 bis 6, **dadurch gekennzeichnet, dass** das anionische Tensid ausgewählt ist unter den (C₁₂-C₁₈) Alkylsulfaten, den (C₁₂-C₁₈) Alkyl-Ether-Sulfaten mit 2 bis 4 Mol Alkylenoxiden bei (C₂-C₄), den Alkylsulfosuccinaten, den (C₈-C₁₆) Alkylbenzensulfonaten.

8. Zusammensetzung zur Herstellung einer dekontaminierenden und schäumenden wässerigen Lösung nach einem der Ansprüche 1 bis 7.

9. Zusammensetzung nach Anspruch 8, **dadurch gekennzeichnet, dass** sie in getrennten Verpackungen I und II enthält:
- den organischen oder mineralischen wasserlöslichen peroxidativen Wirkstoff und eventuell einen Stabilisator dieses wasserlöslichen peroxidativen Wirkstoffes in der Verpackung I,
- die anderen Bestandteile in Verpackung II und eventuell die Gesamtheit oder einen Teil des Wassers, das für die Herstellung der wässerigen Lösung nach einem der Ansprüche 1 bis 7 erforderlich ist.

10. Verwendung der wässerigen Lösung nach einem der Ansprüche 1 bis 7 zur Dekontamination von dispergierenden Systemen und explosiven Wirkstoffen, die chemische oder bakteriologische toxische Wirkstoffe enthalten können.

11. Verfahren zur Dekontamination von Oberflächen, **dadurch gekennzeichnet, dass** es darin besteht:
1) einen Schaumstoff aus einer wässerigen Lösung nach einem der Ansprüche 1 bis 7 zu erzeugen,
2) den Schaumstoff mit der zu dekontaminierenden Oberfläche einige Minuten lang in Kontakt zu bringen,
3) die Ausflüsse nach der Verflüssigung des Schaumstoffes aufzufangen.

12. Verfahren zur Zerstörung von explosiven Stoffen, die chemische oder bakteriologische toxische Wirkstoffe enthalten können, **dadurch gekennzeichnet, dass** es darin besteht:
1) einen Schaumstoff aus einer wässerigen Lösung nach einem der Ansprüche 1 bis 7 zu erzeugen,
2) die zu zerstörenden explosiven Stoffe in einem beweglichen Behälter zu verfeinern,
3) den in Schritt 1) hergestellten Schaumstoff in dem Behälter zu zerstäuben,
4) die zu zerstörenden explosiven Stoffe anzuzünden,
5) die Ausflüsse nach der Verflüssigung des Schaumstoffes aufzufangen.

## Claims

1. A foaming decontaminating aqueous solution, **characterized in that** it comprises, by weight relative to the total weight of the composition:
- 0.1% to 20% of an organic or mineral water-soluble peroxidizing agent;
- 0.05% to 20% of an anionic surfactant;
- 0.1% to 20% of an amine oxide;
- 0 to 5% of a stabilizer for the peroxidizing agent;
- 0 to 10% of an amphoteric surfactant;
- 0 to 15% of an alkaline buffer; and
- the remainder to 100% being water;
said organic or mineral water-soluble peroxidizing agent being chosen from:
- the organic peracids of general formula R'-CO₃H in which R' is a linear or branched alkyl group containing from 1 to 4 carbon atoms and preferably from 1 to 3 carbon atoms and alkali metal, alkaline-earth metal, ammonium or hydroxylamine salts thereof;
- the organic peracids of general formula HO₃C-R'-CO₃H in which R' is a linear or branched alkylene group of 1 to 4 carbon atoms and preferably of 1 to 3 carbon atoms, and the alkali metal, alkaline-earth metal, ammonium or hydroxylamine salts thereof;
- the organic peracids of general formula R"-(Φ) (CO₃H)ₙ(CO₂H)ₘ in which R" is a hydrogen or a linear or branched alkyl group of 1 to 4 carbon atoms, (Φ) is a benzene ring, n = 1 or 2, m = 0 or 1, and the alkali metal, alkaline-earth metal, ammonium or hydroxylamine salts thereof;
- the mineral peroxides chosen from H₂O₂ adducts, persalts, mineral peracids and alkali metal salts thereof,
- mixtures of said peroxidizing agents;
the pH of said aqueous solution being between 7 and 11.

2. The solution as claimed in claim 1, **characterized in that** the pH is between 8.0 and 9.5.

3. The solution as claimed in either of claims 1 and 2, **characterized in that** it comprises, by weight relative to the total weight of the composition:
- 0.3% to 5% of an organic or mineral water-soluble peroxidizing agent;
- 0.1% to 5% of an anionic surfactant;
- 0.5% to 4% of an amphoteric surfactant;
- 0.1% to 5% of an amine oxide;
- 0.1% to 2% of a stabilizer for the peroxidizing agent;
- 0 to 10% of an alkaline buffer; and
- the remainder to 100% being water.

4. The solution as claimed in any one of claims 1 to 3, **characterized in that** the amine oxide is chosen from: 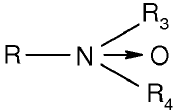 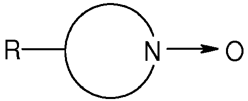 in which:
- R is a linear or branched alkyl or alkenyl group containing 8 to 22 carbon atoms, optionally interrupted with a group -O-C(=O); -O-; -O-NH₂- or -O-NR₁R₂-;
- R₃ and R₄, which may identical or different, represent a C₁-C₄ alkyl or a group of formula [(CH₂)ₘ-O]ₙ-H in which m and n are between 1 and 4;
- 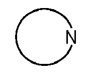 is a heterocycle containing 2 to 6 carbon atoms and at least one nitrogen atom.

5. The solution as claimed in claim 4, **characterized in that** the amine oxide is:
- N-lauryldimethylamine oxide;
- laurylpyridine oxide;
- N-cetyldiethylamine oxide;
- octylpyridine oxide;
- N-myristylbis(2-hydroxyethyl)amine oxide.

6. The solution as claimed in claim 1, **characterized in that** the peroxidizing agent is:
- peracetic acid, sodium peracetate, perpropionic acid, performic acid or potassium perpropionate;
- magnesium monoperoxyphthalate hexahydrate (MPPM), perphthalic acid or perbenzoic acid;
- sodium caroate or sodium percarbonate.

7. The solution as claimed in any one of claims 1 to 6, **characterized in that** the anionic surfactant is chosen from (C₁₂-C₁₈)alkyl sulfates, (C₁₂-C₁₈)alkyl ether sulfates containing 2 to 4 mol of a (C₂-C₄)alkylene oxide; alkylsulfosuccinates; (C₈-C₁₆)alkylbenzensulfonates.

8. A composition for preparing a foaming decontaminating aqueous solution as claimed in any one of claims 1 to 7.

9. The composition as claimed in claim 8, **characterized in that** it comprises, in separate packagings I and II:
- the organic or mineral water-soluble peroxidizing agent and, optionally, a stabilizer for this water-soluble peroxidizing agent, in packaging I;
- the other constituents in packaging II, and optionally all or some of the water, necessary for preparing the aqueous solution as claimed in any one of claims 1 to 7.

10. The use of the aqueous solution as claimed in any one of claims 1 to 7 for decontaminating dispersant systems and explosive agents liable to contain chemical or bacteriological toxic agents.

11. A process for decontaminating surfaces, **characterized in that** it consists in:
1) generating a foam from an aqueous solution as claimed in any one of claims 1 to 7;
2) placing said foam in contact with the surface to be decontaminated for a few minutes;
3) recovering the effluents after the foam has liquefied.

12. A process for destroying explosives liable to contain chemical or bacteriological toxic agents, **characterized in that** it consists in:
1) generating a foam from an aqueous solution as claimed in any one of claims 1 to 7;
2) confining the explosives to be destroyed in a mobile chamber;
3) spraying the foam obtained in step 1) into said chamber;
4) igniting said explosives to be destroyed;
5) recovering the effluents after the foam has liquefied.
